# EUROPEAN PATENT APPLICATION

(11) **EP 4 659 694 A1**
(43) Date of publication of application: **10.12.2025**
(21) Application number: 25181227.7
(22) Date of filing: 06.06.2025
(51) Int. Cl.: A61B 18/14, A61B 90/00

(54) **ABLATION TAGS INDICATOR**

(30) Priority: 07.06.2024 US 202418736736
(71) Applicant: Biosense Webster (Israel) Ltd., 2066717 Yokneam (IL)
(72) Inventor: SEGEV, Meytal, 2066717 Yokneam (IL); ZIDAN, Layla Saleem, 2066717 Yokneam (IL); KEREN TAL, Dor Zeev, 2066717 Yokneam (IL); BEN NATAN, Alon, 2066717 Yokneam (IL); YANOVICH, Nir, 2066717 Yokneam (IL); KASSAR, Lotan Dvir, 2066717 Yokneam (IL)
(74) Representative: Carpmaels & Ransford LLP

(57) **Abstract**

A method includes receiving or generating an anatomical map of wall tissue of at least a portion of a cardiac chamber, the map superimposed with ablation tags at locations of ablated wall tissue. Upon placing an ablation catheter having one or more electrodes in a vicinity of the wall tissue, at least one electrode among the one or more electrodes is identified, that violates a location proximity criterion to one or more of the ablation tags. The identified at least one electrode is graphically encoded. The anatomical map showing the graphically encoded at least one electrode is displayed to a user.

## Description

### FIELD OF THE DISCLOSURE

This disclosure relates generally to cardiac ablation, and specifically to a system and method for real-time planning and monitoring of cardiac ablation using an anatomical map.

### BACKGROUND OF THE DISCLOSURE

Automatically updatable graphical representation of catheter electrodes was previously proposed in patent literature. For example, U.S. patent Application 2021/0082157 describes an ablation catheter with an expandable balloon or a basket, a plurality of electrodes disposed on the surface, and a graphical user interface (GUI) of a computer system. The GUI includes a plurality of electrode representations, a first sector color-coded plot of at least one of the plurality of electrodes, a second sector color-coded plot of at least one of the plurality of electrodes, a third sector color-coded plot of at least one of the plurality of electrodes, and a processor configured to receive data on the inputs based on at least one of the plurality of electrodes, change an appearance of at least one of the first, second sector, and the third sector based on the received data, and update the color-coded plot of the first input.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Figs. 2A and 2B depict two real-time instances of an anatomical map, superimposed with ablation tags and graphical encodings of catheter electrodes, for identifying electrodes suited to ablate wall tissue, in accordance with an example of the present disclosure;
Fig. 3 is a schematic illustration of a graphical user interface (GUI) used for graphically encoding the ablation electrodes of Fig. 2, in accordance with some examples of the present disclosure;
Fig. 4 shows several ablation catheter assemblies with one or more electrodes that may be highlighted by the disclosed technique, in accordance with examples of the present disclosure; and
Fig. 5 is a flow chart that schematically illustrates a method for real-time planning and monitoring of ablation by graphically encoding ablation electrodes, in accordance with an example of the present disclosure.

### DETAILED DESCRIPTION OF EXAMPLES

### OVERVIEW

In a cardiac ablation procedure, such as pulmonary vein isolation (PVI) to treat atrial fibrillation, a physician ablates tissue in a specific anatomical region, (e.g., over an entire circumference of an ostium of a PV). The ablation, such as one using a pulsed-field ablation (PFA) technique, may require several iterations to fully cover the entire circumference of the ostium. Each iteration requires moving a multi-electrode ablation catheter to areas that are either still insufficiently ablated, or not ablated at all.

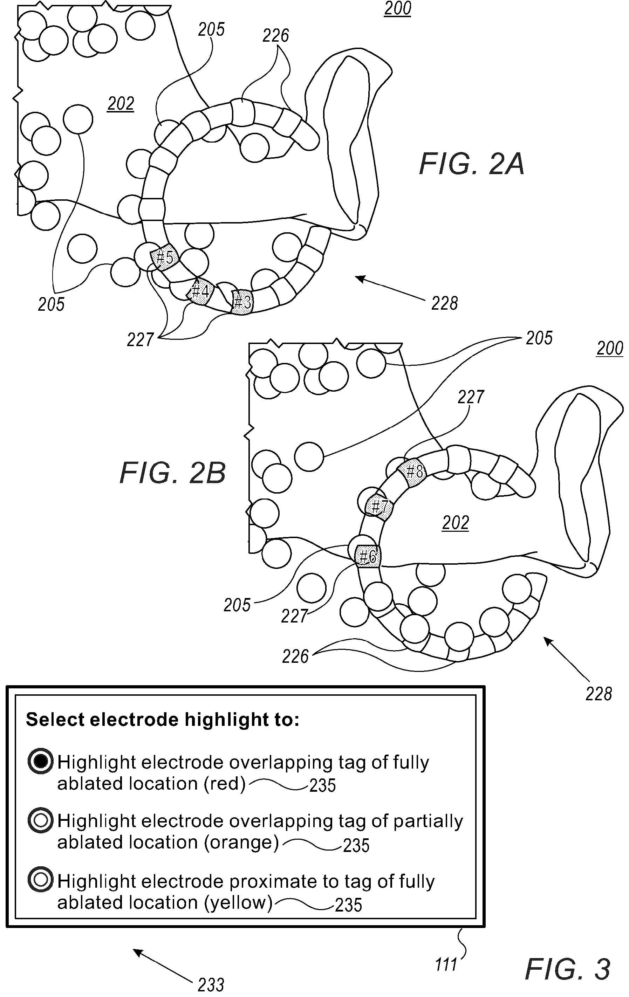

In this disclosure it is assumed that the physician is aided by an anatomical 3D map that displays ablation tags (seen in Fig. 2) in relation to a surface of a 3D anatomical map, including ones initially positioned in 3D space sufficiently near the anatomical 3D map surface and accurately projected onto the surface. The ablation tags mark surface tissue locations that were ablated.

The ablation tags were found by the authors to sometimes be less helpful to the clinician than might otherwise be expected. A physician attempting to complete the ablation may still face several challenges using the map, including (a) difficulty in closing ablation gaps, (b) difficulty aligning a visualized 3D location with a previous ablation location, and (c) preventing repetitive ablations on the same tissue location.

To aid a physician cope with the challenges listed above, e.g., to make the correct decisions about where and how to perform further ablations, some examples of the present disclosure that are described hereinafter provide real-time updatable visualizations of a catheter's electrodes based on the ablation tags and based on violating a location proximity criterion to one or more of the ablation tags.

In one example, a processor applying the disclosed technique identifies at least one electrode among the one or more electrodes that violates a location proximity criterion to one or more of the ablation tags. The processor graphically encodes the identified at least one electrode. For example, the processor highlights every electrode of the multi-electrode catheter when the current electrode location violates the location proximity criterion by coinciding with (e.g., overlaps) an existing ablation tag location.

To find if an electrode location violates the location proximity criterion, the processor uses an algorithm to calculate the distance of each electrode location (e.g., its center of gravity) from the ablation tag's center location, and then compares the distances to a predetermined threshold. When the distance is smaller than the threshold, the relevant electrode is considered to be overlapping the tag and the electrode is highlighted, e.g. colored red.

The user may then relocate the catheter to prevent over-ablating the same location, or may decide to deactivate certain catheter electrodes for the next iteration of the application of PFA.

In an example, the user configures the following (next) ablation by choosing only some of the electrodes of the multi-electrode catheter to be used for applying ablation. In this case, only electrodes belonging to the selection made by the user will show any graphical indication. This step minimizes visual clutter that may be caused by unnecessarily highlighting inactive (e.g., defected, not selected by user for clinical reason) electrodes.

In another example of the disclosed real-time visualization technique, a processor receives or generates an anatomical map of wall tissue of at least a portion of a cardiac chamber, the map superimposed with ablation tags at locations of ablated wall tissue, with the ablation tags graphically encoded according to respective levels of wall tissue ablation. For example, the map would show an ablation tag of one form (e.g., color, shape) over a fully ablated location, and another form of ablation tag over a partially ablated location. When the electrode distance from the ablation tag in a fully ablated location is smaller than the threshold, the relevant electrode is highlighted in one way (e.g. colored red). If the ablation tag is placed over a partially ablated location, the relevant electrode is highlighted in a different way (e.g. colored orange). This method of electrode highlighting allows a physician to complete the ablation while avoiding over-ablating a same tissue location.

In yet another example, violation of the location proximity criterion is defined more broadly. In this case, the electrode location violates the location proximity criterion by being located sufficiently close but not overlapping the ablation tag location (e.g., the distance is above the above threshold that can be redesignated as a first threshold, but below a second threshold). In such case the processor applying the disclosed technique graphically encodes (e.g., highlights) the electrode in another distinctive way (e.g., colors the electrode yellow). When the electrode distance becomes smaller than the first threshold, the relevant electrode is colored as above (e.g., red or orange). This sort of highlighting assists a physician to navigate the catheter to a previously ablated location to, for example, check the level of ablation therein and act accordingly.

In some examples, shown in Fig. 3, a graphical user interface (GUI), provided by the disclosed technique, allows a user to modify the location proximity criterion. For example, the GUI allows to graphically encode one or more electrodes of a catheter according to (i) location proximity of the catheter electrodes to ablation tags, and, (ii) ablation level the tag represents, such as indicating wall tissue location being fully ablated and/or partially ablated (e.g., incompletely ablated). For example, if all checkboxes of such a GUI are selected, the electrode color can vary, depending on electrode location, from a default color (e.g., light grey) to yellow, and then to orange or red.

### SYSTEM DESCRIPTION

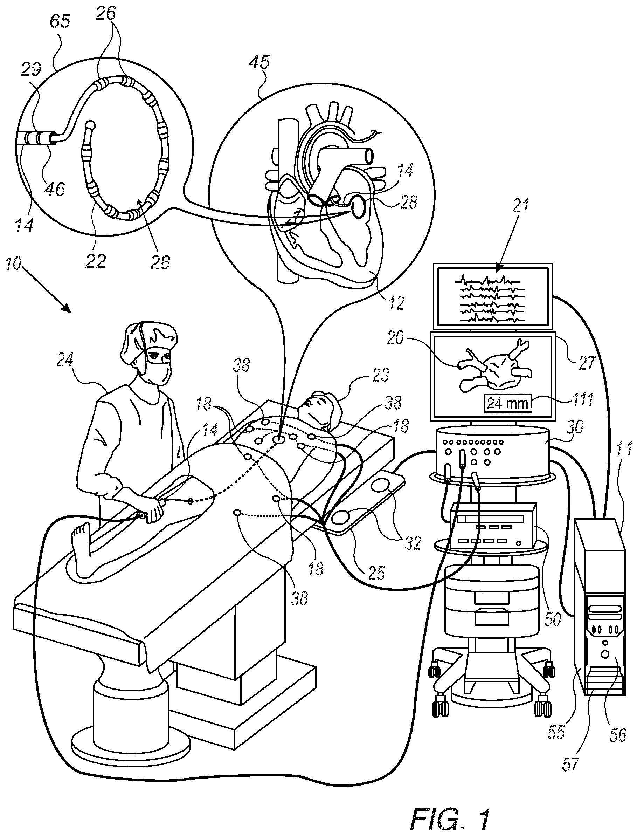

Fig. 1 is a schematic, pictorial illustration of a catheter-based electroanatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example catheter 14, illustrated herein, is configured for sensing bipolar electrograms and pulsed field ablation (PFA). Physician 24 brings a distal tip 28 (also called hereinafter "distal end assembly 28") of catheter 14 into contact with the heart wall for ablating a target site in heart 12.

As seen in inset 65, catheter 14 is an exemplary catheter that includes a lasso distal end assembly 28, including one, and preferably multiple, electrodes 26 optionally distributed over a curved spline 22. Catheter 14 may additionally include a position sensor 29, embedded in or near distal tip 28 on a shaft 46 of catheter 14, to track the position and orientation of distal tip 28. Optionally, and preferably, position sensor 29 is a magnetic-based position sensor including three magnetic coils for sensing three-dimensional (3D) position and orientation.

The magnetic-based position sensor 29 may be operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by magnetic-based position sensor 29. Details of the magnetic-based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

System 10 may include an ablation energy generator 50 adapted to conduct ablative energy to one or more electrodes at a distal tip of a catheter configured for ablation. The energy produced by ablation energy generator 50 may include, but is not limited to, radiofrequency (RF) energy or pulse field (PF) energy, including monopolar or bipolar high-voltage DC pulses, to be used to effect irreversible electroporation (IRE) or combinations thereof.

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

In the disclosed example, processor 56 runs an algorithm that highlights every electrode 26 of the multi-electrode catheter 14 when the current electrode 26 location violates the location proximity criterion by, for example, being in a defined location proximity to the ablation tags but not overlapping any of the ablation tags. The real-time highlighting can further depend on the existing level of ablation and on the ablation electrode's further location proximity (e.g., overlapping), as shown in Fig 3.

In some examples, processor 56 typically comprises a general-purpose computer programmed in software to carry out the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as a basket catheter.

### GRAPHICAL ENCODING OF CATHETER ELECTRODES IN RELATION TO ABLATION TAGS

Figs. 2A and 2B depict two real-time instances of an anatomical map 202 superimposed with ablation tags 205 and graphical encodings 227 of catheter electrodes 226, for identifying electrodes 226 suited to ablate wall tissue, in accordance with an example of the present disclosure. Ablation tags 205 are superimposed on a 3D anatomical model which additionally displays another electroanatomical (EA) parameter, e.g., a late activation time (LAT) map.

In Figs. 2A and 2B, an ablation catheter, such as a loop catheter assembly 228 (e.g., VARIPULSE^{®} catheter provided by Biosense Webster) carrying electrodes 226, is shown located in vicinity to wall tissue to finalize an ablation. The processor graphically encodes (227) only those electrodes 226 that violate the location proximity criterion to the ablation tags 205 (e.g., based on an electrode-tag location proximity calculation described above). An electrode 226 retains its default appearance (e.g., colored pale grey) when located farther from any ablation tag 205.

Figs. 2A and 2B capture different real-time locations of the VARIPULSE^{®} catheter assembly 228, the two figures differing only by the identity of highlighted catheter electrodes in relation to ablation tags 205. In the instance shown in Fig. 2A, electrodes #3, #4, and #5 are highlighted to warn the user not to activate them. Similarly, in the instance shown in Fig. 2B, electrodes #6, #7, and #8 are highlighted to warn the user not to activate them.

Finally, different types of graphical encodings 227 may be applied to electrode 226, such as colors or patterns. In one example, the disclosed technique can apply color encoding (e.g., red, orange, yellow) to indicate (i) an electrode overlapping an ablation tag indicating a fully ablated location, (ii) an electrode overlapping an ablation tag indicating a partially ablated location, or (iii) an electrode located proximate to an ablation tag indicating a fully ablated location but not overlapping the tag, respectively. In another example, the disclosed technique can highlight electrodes with different pattern arrays, such as using the symbols #, //, \\, =, or ∥, among others.

Fig. 3 is a schematic illustration of a graphical user interface (GUI) 111 used for graphically encoding the ablation electrodes 226 of Fig. 2, in accordance with some examples of the present disclosure.

A user of GUI 111 can select to graphically encode the ablation electrodes 226 of Fig. 2 according to the level of existing ablation and/or according to electrode-tag location proximity. To this end, the user can select one or more of checkboxes 233 on the GUI, e.g., according to user preferences.

The user can use GUI 111 configured to allow the user to modify the location proximity criterion. In the example shown in Fig. 3, the selected to highlight (235) only electrodes that overlap ablation tags representing fully ablated locations, as shown in Figs. 2A and 2B.

The user may select all of checkboxes 233 on the GUI to highlight (235) as in the following example: red highlighted electrodes overlapping ablation tags represent fully ablated locations; orange highlighted electrodes overlapping ablation tags represent partially ablated locations; yellow highlighted electrodes located sufficiently proximate to ablation tags represent fully ablated locations.

The user can replace the type of graphical encodings 235, to, for example, swap one set of highlighting colors for another, or use a set of patterns instead of colors.

GUI 111 us brought by way of example. In another example, the GUI includes a checkbox that the user can select in order to highlight only electrodes belonging to a group of electrodes that the user chosen for applying any subsequent ablation.

### ABLATION CATHETERS USED WITH GRAPHICAL ENCODING OF ELECTRODE TECHNIQUE

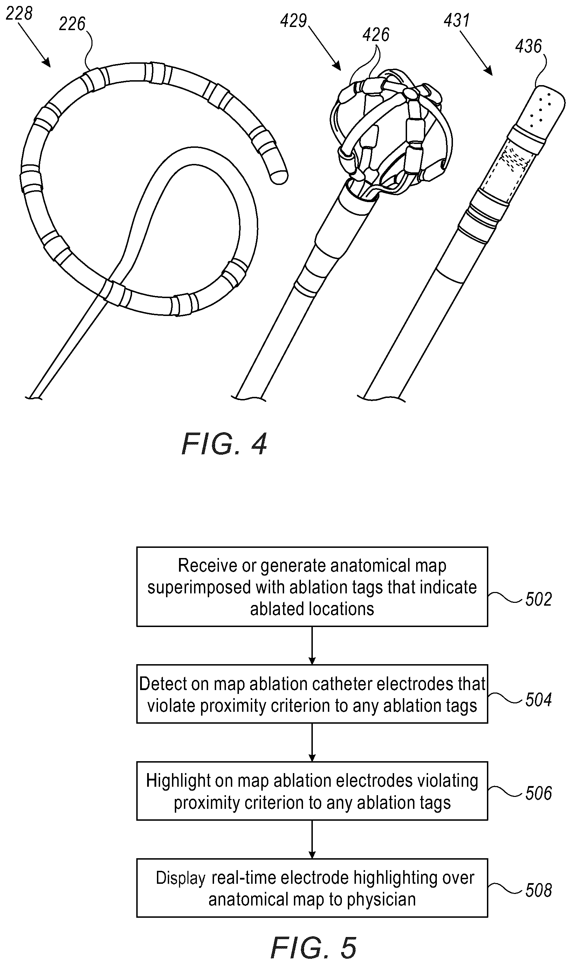

Fig. 4 shows several ablation catheter assemblies (228, 429, 431) with one or more respective electrodes (226, 426, 436) that the disclosed technique may highlight in accordance with examples of the present disclosure. Assembly 228 is the loop catheter described in Figs. 1 and 2 above. Assembly 426 is in the shape of a basket with its electrodes 436 disposed over the basket splines.

Tip catheter 436 has only one ablation electrode 436 that can be highlighted according to user selection in GUI 111 of Fig. 3. If the user selects all checkboxes 233, then electrode 436, as presented over map 202, changes colors from red to orange to yellow according to location proximity to ablation tags and level of previous ablation represented by the tag. When the electrode is far enough from any ablation tag it maintains its default appearance (e.g., colored grey).

### METHOD OF GRAPHICAL ENCODING OF CATHETER ELECTRODES IN RELATION TO ABLATION TAGS

Fig. 5 is a flow chart that schematically illustrates a method for real-time planning and monitoring ablation by graphically encoding ablation electrodes, in accordance with an example of the present disclosure. The algorithm, according to the presented example, carries out a process that begins with processor 56 receiving or generating an anatomical map 202 superimposed with ablation tags 205 that indicate wall tissue locations already ablated, at an ablation map receiving step 502.

Next, as the physician brings a single-electrode (431) or multi-electrode ablation catheter (228, 429) into the location proximity of wall tissue, the processor detects the one or more catheter electrodes violates the location proximity criterion to any ablation tag 205 at a pre-ablating step 504.

In response to detecting electrode-tag overlap, the processor highlights any ablation electrode violating the location proximity criterion, as seen in Fig. 2, in highlighting electrodes step 506.

At map displaying step 508, the processor displays the user with a map with real-time highlighted electrodes to guide the physician about where to apply the upcoming ablation and/or which electrode to use.

### EXAMPLES

### Example 1

A method includes receiving or generating an anatomical map (200) of wall tissue of at least a portion of a cardiac chamber, the map (200) superimposed with ablation tags (205) at locations of ablated wall tissue. Upon placing an ablation catheter (14) having one or more electrodes (26, 226) in a vicinity of the wall tissue, at least one electrode (226) among the one or more electrodes is identified, that violates a location proximity criterion to one or more of the ablation tags (205). The identified at least one electrode (226) is graphically encoded (227). The anatomical map (200) showing the graphically encoded (227) at least one electrode (226) is displayed (27) to a user.

### Example 2

The method according to example 1, wherein the ablation tags (205) are graphically encoded according to a level of ablation, and wherein graphically encoding (227) the electrode comprise graphically encoding the electrode according to graphic encoding of the ablation tags (205) for which the location proximity criterion is violated.

### Example 3

The method according to ant of examples 1 and 2, wherein graphically encoding (227) the electrode (226) comprises encoding the electrode differently when the location proximity criterion is violated with respect to an ablation tag (205) indicative of a fully ablated location, and when the location proximity criterion is violated with respect to an ablation tag (205) indicative of a partially ablated location.

### Example 4

The method according to any of examples 1 through 3, wherein encoding (227) the electrode (226) differently comprises highlighting the electrode (226) with different colors depending on whether the location proximity criterion is violated with respect to an ablation tag (205) indicative of a fully ablated location, or with respect to an ablation tag (205) indicative of a partially ablated location.

### Example 5

The method according to any of examples 1 through 4, wherein the location proximity criterion is violated when an electrode (226) is in a defined location proximity to the ablation tags (205) but does not overlap any of the ablation tags (205).

### Example 6

The method according to claim any of examples 1 through 5, wherein graphically encoding (227) the at least one electrode (226) is performed in real-time, as the ablation catheter (14) is navigated in the cardiac chamber.

### Example 7

The method according to any of examples 1 through 6, and comprising providing a graphical user interface (GUI) (111) configured to allow the user to modify the location proximity criterion.

### Example 8

8. The method according to any of examples 1 through 6, wherein graphically encoding (227) the at least one electrode (227) comprises graphically encoding only electrodes (226) belonging to a group of electrodes selected by the user.

### Example 9

The method according to any of examples 1 through 8, wherein the anatomical map (200) is an electroanatomical (EA) map.

### Example 10

A system includes a display device (27) and a processor (56). The processor is configured to (i) receive or generate an anatomical map (200) of wall tissue of at least a portion of a cardiac chamber, the map superimposed with ablation tags (205) at locations of ablated wall tissue, (ii) upon placing an ablation catheter (14) having one or more electrodes (26, 226) in a vicinity of the wall tissue, identify at least one electrode (226) among the one or more electrodes that violates a location proximity criterion to one or more of the ablation tags (205), (iii) graphically encode (227) the identified at least one electrode (226), and (iv) display on the display device (27) the anatomical map (200), showing the graphically encoded (227) at least one electrode (226), to a user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system, comprising:
a display device (27); and
a processor (56), which is configured to:
receive or generate an anatomical map (200) of wall tissue of at least a portion of a cardiac chamber, the map (200) superimposed with ablation tags (205) at locations of ablated wall tissue;
upon placing an ablation catheter (14) having one or more electrodes (26, 226) in a vicinity of the wall tissue, identify at least one electrode (226) among the one or more electrodes that violates a location proximity criterion to one or more of the ablation tags (205);
graphically encode (227) the identified at least one electrode (226); and
display on the display device (27) the anatomical map (200), showing the graphically encoded (227) at least one electrode (226), to a user.

2. The system according to claim 1, wherein the ablation tags (205) are graphically encoded (227) according to a level of ablation, and wherein graphically encoding (227) the electrode (226) comprise graphically encoding (227) the electrode (226) according to graphic encoding of the ablation tags (205) for which the location proximity criterion is violated.

3. The system according to any one of claims 1-2, wherein the processor (56) is configured to graphically encode (227) the electrode (226) by encoding the electrode differently when the location proximity criterion is violated with respect to an ablation tag (205) indicative of a fully ablated location, and when the location proximity criterion is violated with respect to an ablation tag (205) indicative of a partially ablated location.

4. The system according to any one of claims 2-3, wherein the processor (56) is configured to encode the electrode (226) differently by highlighting the electrode (226) with different colors depending on whether the location proximity criterion is violated with respect to an ablation tag (205) indicative of a fully ablated location, or with respect to an ablation tag (205) indicative of a partially ablated location.

5. The system according to claim 1, wherein the location proximity criterion is violated when an electrode (226) is in a defined location proximity to the ablation tags (205) but does not overlap any of the ablation tags (205).

6. The system according to claim 1, wherein the processor (56) is configured to graphically encode (227) the at least one electrode (226) in real-time, as the ablation catheter (14) is navigated in the cardiac chamber.

7. The system according to any one of claims 1-6, wherein the processor (56) is further configured to provide a graphical user interface (GUI) configured to allow the user to modify the location proximity criterion.

8. The system according to any one of claims 1-7, wherein the processor (56) is configured to graphically encode (227) the at least one electrode (226) by graphically encoding (227) only electrodes belonging to a group of electrodes selected by the user.

9. The system according to any one of claims 1-8, wherein the anatomical map (200) is an electroanatomical (EA) map.
